(19) Europäisches Patentamt / European Patent Office / Office européen des brevets

(11) **EP 4 342 443 A1**

(12) **EUROPEAN PATENT APPLICATION**
published in accordance with Art. 153(4) EPC

(43) Date of publication:
**27.03.2024 Bulletin 2024/13**

(21) Application number: **22804651.2**

(22) Date of filing: **16.05.2022**

(51) International Patent Classification (IPC):
*A61K 8/02* (2006.01)      *A61K 8/19* (2006.01)
*A61K 8/34* (2006.01)      *A61K 8/44* (2006.01)
*A61K 8/46* (2006.01)      *A61K 8/73* (2006.01)
*A61K 8/81* (2006.01)      *A61Q 19/00* (2006.01)

(52) Cooperative Patent Classification (CPC):
**A61K 8/02; A61K 8/19; A61K 8/34; A61K 8/44;
A61K 8/46; A61K 8/73; A61K 8/81; A61Q 19/00**

(86) International application number:
**PCT/JP2022/020410**

(87) International publication number:
**WO 2022/244740 (24.11.2022 Gazette 2022/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.05.2021 JP 2021086072
18.10.2021 JP 2021170067**

(71) Applicant: **Kao Corporation
Tokyo 103-8210 (JP)**

(72) Inventor: **SONODA, Junko
Tokyo 131-8501 (JP)**

(74) Representative: **Hoffmann Eitle
Patent- und Rechtsanwälte PartmbB
Arabellastraße 30
81925 München (DE)**

(54) **AEROSOL COSMETIC**

(57)      An aerosol cosmetic comprising a stock solution comprising the following components (A), (B), and (C): (A) an anionic surfactant; (B) an anionic polymer; and (C) a cationic polymer having a cationic charge density of 5.0 meq/g or less, a mass ratio of the component (A) to a total of the components (B) and (C), (A)/((B)+(C)), being 6 or more, and (D) carbon dioxide gas.

**Description**

Field of the Invention

The present invention relates to an aerosol cosmetic.

Background of the Invention

Skin troubles such as dark circles under the eyes, and dullness are considered to be caused by a poor blood flow, and in order to improve these troubles, cosmetics utilizing the effect of carbon dioxide gas have been studied.

For example, Patent Literature 1 describes a foamy skin coating agent comprising N-acylamino acid or a salt thereof, a (meth)acrylic acid polymer, a polysaccharide polymer, carbon dioxide, and hydrocarbon having 3 to 5 carbon atoms, Patent Literature 2 describes a foamy skin coating agent comprising a specific surfactant, a (meth)acrylic acid polymer, a polysaccharide polymer, carbon dioxide, and hydrocarbon having 3 to 5 carbon atoms, in both of which a good blood circulation promotion action is described.

(Patent Literature 1) JP-A-2010-248098
(Patent Literature 2) JP-A-2011-84490

Summary of the Invention

The present invention relates to an aerosol cosmetic containing a stock solution containing:
the following components (A), (B), and (C),

(A) an anionic surfactant;
(B) an anionic polymer; and
(C) a cationic polymer having a cationic charge density of 5.0 meq/g or less,
a mass ratio of the component (A) to a total of the components (B) and (C), (A)/((B)+(C)), is 6 or more, and
(D) carbon dioxide gas.

The present invention also relates to a method for producing the aerosol cosmetic including the following steps 1 to 3:

step 1: a step of stirring components including the components (A) and (B), and heating them to 50°C or more to mix the components,
step 2: a subsequent step of adding the component (C), and stirring and mixing the components to prepare a stock solution, and
step 3: a step of cooling the stock solution obtained in the step 2, then filling the stock solution into a pressure resistant container, hermetically sealing the container, and thereafter filling the component (D) under pressure.

The conventional aerosol cosmetics containing carbon dioxide gas had ununiform texture of a foam discharged from a container, and poor foam retention, thereby posing a problem of lastingness during massage.

The present inventor found that an aerosol cosmetic with uniform texture of the discharged foam, good foam retention, and excellent lastingness during massage can be obtained when an anionic surfactant is used in a specific ratio in relative to an anionic polymer and a cationic polymer having a specific charge density.

The aerosol cosmetic of the present invention has uniform texture of the discharged foam, good foam retention, and excellent lastingness during massage. A feel of moist skin after washing also remains.

The anionic surfactant of the component (A) used in the present invention are those used for typical cosmetics, and examples include fatty acids having 12 to 22 carbon atoms such as sodium laurate, potassium palmitate, and arginine stearate, or salts thereof; alkyl sulfate esters having 12 to 22 carbon atoms such as sodium lauryl sulfate, and potassium lauryl sulfate, or salts thereof; polyoxyethylene alkyl ether sulfates such as sodium polyoxyethylene(2) lauryl sulfate, or salts thereof; polyoxyethylene alkyl ether phosphates having 12 to 22 carbon atoms such as sodium polyoxyethylene oleyl ether phosphate, sodium polyoxyethylene stearyl ether phosphate, and sodium polyoxyethylene lauryl ether phosphate, or salts thereof; dialkyl sulfosuccinic acids having 12 to 24 carbon atoms such as sodium di-2-ethylhexylsulfosuccinate, or salts thereof; N-alkyloyl methyl taurines having 12 to 22 carbon atoms such as sodium N-stearoyl-N-methyltaurine, or salts thereof; N-acyl glutamic acids having 12 to 22 carbon atoms such as sodium dilauroyl glutamate, monosodium N-lauroyl glutamate, sodium N-stearoyl-L-glutamate, arginine N-stearoyl-L-glutamate, sodium N-stearoyl glutamate, and sodium N-myristoyl-L-glutamate, or salts thereof. Of these, polyoxyethylene alkyl ether sulfates, or salts thereof, and N-acyl glutamic acids having 12 to 22 carbon atoms, or salts thereof are preferably, and polyoxyethylene lauryl ether sulfates, or salts thereof, and N-acyl glutamic acid of coconut oil fatty acid, or a salt thereof are more preferable, from a viewpoint of forming a fine foam when discharging carbon dioxide gas, and enhancing the foam retention.

Examples of these salts include alkali metals such as sodium, and potassium; alkali earth metals such as calcium, and magnesium; ammonium; organic ammonium derived from alkanolamine such as monoethanolamine, diethanolamine, and triethanolamine; trishydroxymethyl aminomethane; basic amino acids such as L-arginine, with sodium being preferable.

The anionic surfactant of the component (A) can be used singly, or in combination of two or more surfactants, and the content in the stock solution is preferably 1 mass% or more, more preferably 3 mass% or more, further more preferably 5 mass% or more, and preferably 40 mass% or less, more preferably 25 mass% or less, and further preferably 15 mass% or less, from a viewpoint of forming a fine foam when discharging carbon dioxide gas, and enhancing the foam retention. Additionally, the content of the component (A) in the stock solution is preferably from 1 to 40 mass%, more preferably from 3 to 25 mass%, and further preferably from 5 to 15 mass%.

The anionic polymer of the component (B) can be those used for typical cosmetics, and examples include acrylic acid polymers such as polyacrylic acid, polymethacrylic acid, acrylic acid/(meth)acrylic acid alkyl copolymers, carboxyvinyl polymers, and polyoxyethylene alkyl ether-modified acrylic acid copolymers; and carboxymethyl cellulose, carrageenan, xanthan gum, polystyrene sulfonate, agar, ghatti gum, karaya gum, pectin, alginate salt, and hyaluronic acid or alkali metal salts thereof.

Of these, acrylic acid polymers are preferable, acrylic acid/(meth)acrylic acid alkyl copolymers, carboxyvinyl polymers, and polyoxyethylene alkyl ether-modified acrylic acid copolymers are more preferable, and it is further preferable to contain at least one selected from the group consisting of (acrylates/ ($C_{10-30}$) alkyl (meth)acrylate) copolymers, carboxyvinyl polymers, and (acrylates/steareth-20 methacrylate) copolymers, from a viewpoint of preventing foams from colliding, and retaining carbon dioxide gas.

The (acrylates/ ($C_{10-30}$) alkyl (meth) acrylate) copolymer herein refers to a copolymer of $C_{10-30}$ alkyl acrylate and acrylic acid, methacrylic acid, or a lower alkyl ester thereof, and crosslinked with allyl ether of sucrose or allyl ether of pentaerythritol. Usable commercial products are Pemulen TR-1, Pemulen TR-2, Carbopol ETD2020, Carbopol 1342, Carbopol 1382 (all from Lubrizol Advanced Materials), and the like. Usable commercial products for the (acrylates/steareth-20 methacrylate) copolymer include Aculyn 22 (The Dow Chemical Company), and the like.

The acrylic acid polymer is preferably used as a water soluble or water dispersible salt, using, as a neutralizer, a base like an alkali metal hydroxide such as potassium hydroxide, and sodium hydroxide.

One or more of the component (B) can be used, and the content in the stock solution is preferably 0.01 mass% or more, more preferably 0.02 mass% or more, further more preferably 0.04 mass% or more, and preferably 2 mass% or less, more preferably 1.5 mass% or less, and further preferably 1 mass% or less, from a viewpoint of preventing foams from colliding, and retaining carbon dioxide gas. The content of the component (B) in the stock solution is preferably from 0.01 to 2 mass%, more preferably from 0.02 to 1.5 mass%, and further preferably from 0.04 to 1 mass%.

The component (C) is a cationic polymer having a cationic charge density of 5.0 meq/g or less.

It is considered that such a cationic polymer having a specific charge density aggregates with the anionic polymer of the component (B) and forms in the aerosol cosmetic a structure called a polyion complex (hereinafter, PIC) containing the anionic surfactant of the component (A), water, and other components (for example, the polyhydric alcohol to be described). PIC adsorbs onto the film of foams discharged and stabilizes the foams, whereby it is presumed that the aerosol cosmetic containing PIC provides the foam having uniform foam texture, good foam retention, and excellent lastingness during massage. Further, it is presumed that when the skin is washed with the foam of the aerosol cosmetic containing PIC, PIC remains on the skin even after the foam is washed off, thereby leaving the skin moist after washing.

The charge density of the component (C) is preferably from 0.8 to 4 meq/g, and more preferably from 1 to 3.3 meq/g, from a viewpoint of allowing PIC to adsorb onto the film of foams and forming dense foams capable of retaining carbon dioxide gas.

The cationic charge density herein refers to the ratio of the number of positive charges on the polymer to the molecular weight of the polymer (the weight of counterion of a cationic group is excluded). When a cationic charge density is multiplied by a polymer molecular weight, the number of sites positively charged in a predetermined polymer chain can be determined. The cationic charge density is further defined as the number of milliequivalent of positive charge (cationic nitrogen atoms) per gram (meq/g) of the polymer.

The value of cationic charge density of the component (C) can be determined in accordance with, for example, the following equation (1).

$$\text{Cationic charge density (meq/g)} = 1 \div (\text{unit molecular weight comprising a cationic nitrogen atom in a cationic polymer}) \times 1000 \quad (1)$$

The measurement method of cationic charge density of the component (C) includes a method of determining a nitrogen content derived from a quaternary nitrogencontaining group in the cationic polymer by Kjeldahl method and then calculating the cationic charge density, and a colloid titration method using an aqueous solution of potassium polyvinyl sulfate, and either method can determine a cationic charge density, but in the present invention the measurement is preferably carried out by Kjeldahl method.

For example, Kjeldahl method can follow The Japanese standards of quasi-drug ingredients 2006, general test methods, Nitrogen Determination, 2nd method. Specifically, 100 mg of the purified and dried component (C) is precisely weighed, 10 mL of sulfuric acid and 1 tablet of a digestion accelerator (Nakayama Rika Seisakusho K.K., KJELTABS) are added thereto to carry out complete digestion using a Kjeldahl digestion system (manufactured by BUCHI, K-432) while increasing temperatures in the order of 250°C for 30 minutes, 300°C for 30 minutes, and 420°C for 80 minutes. After completion of the digestion reaction, 30 mL of ionexchange water is added to a sample, and 40 mL of an aqueous solution of 30% sodium hydroxide is added using an automatic Kjeldahl Distillation/titration Unit (manufactured by BUCHI, K-370) to alkalize, then the ammonia isolated by the distillation operation is collected in an aqueous solution of 1% boric acid and titrated using 0.01 N sulfuric acid (manufactured by Wako Pure Chemical Industries, Ltd., for quantitative analysis) thereby to determine a nitrogen content (mass%) in the component (C).

Specific examples of the component (C) include cationized guar gum; cationized tara gum; cationized locust bean gum; cationized polyvinyl alcohol; cationized cellulose; cationized hydroxyalkyl celluloses such as cationized hydroxyethyl cellulose, and cationized hydroxypropyl cellulose; cationic starch; vinylpyrrolidone/N,N-dimethylaminoethyl methacrylate diethyl sulfate copolymers; N,N-dimethylaminoethyl methacrylate diethyl sulfate/N,N-dimethylacrylamide/polyethylene glycol dimethacrylate copolymers; polydiallyldimethylammonium chloride; diallyldimethylammonium chloride/acrylamide copolymers; hydroxyethyl cellulose/diallyldimethylammonium chloride copolymers; vinylimidazolium trichloride/vinylpyrrolidone copolymers; vinylpyrrolidone/alkylamino(meth)acrylate copolymers; vinylpyrrolidone/alkylamino(meth)acrylate/vinylcaprolacta m copolymers; vinylpyrrolidone/(meth)acrylamidopropyl trimethylammonium chloride copolymers; alkyl acrylamide/(meth)acrylate/alkylaminoalkyl acrylamide/polyethylene glycol (meth)acrylate copolymers; and cationic polymers described in JP-A-S53-139734 and JP-A-S60-36407.

Commercial products usable for the cationic polymer of the component (C) are, for example, as follows.

(Cationized guar gum)
Jaguar C-13S, Jaguar C-14S, Jaguar C-14SK, and Jaguar C-17 (all are manufactured by Solvay Rhodia), and the like
(Cationized tara gum)
CATINAL CTR-100 (manufactured by TOHO Chemical Industry Co., Ltd.), and the like
(Cationized hydroxyethyl cellulose)
Polyquaternium-10 (o-[2-hydroxy-3-(trimethylammonio)propyl]hydroxyethyl cellulose chloride): UCARE polymer JR-400 (manufactured by The Dow Chemical Company), POIZ C-60H (manufactured by Kao Corporation), POIZ C-150L (manufactured by Kao Corporation), Caticello M-80 (manufactured by Kao Corporation), and the like
(Cationized hydroxypropyl cellulose)
Sofcare C-HP2 (manufactured by Kao Corporation), and the like
(Cationized polyvinyl alcohol)
CM318 (manufactured by Kuraray Co., Ltd), and the like
(Vinylpyrrolidone/N,N-dimethylaminoethyl methacrylate diethyl sulfate copolymer)
Polyquaternium-11: GAFQUAT 755N (manufactured by ISP Japan Ltd.), and the like
(N,N-Dimethylaminoethyl methacrylate diethyl sulfate/N,N-dimethylacrylamide/polyethylene glycol dimethacrylate copolymer)
Polyquaternium-52: Sofcare KG-101W-E (manufactured by Kao Corporation), and the like
(Diallyldimethylammonium chloride/acrylamide copolymer)
Polyquaternium-7: MERQUAT 550 (manufactured by Lubrizol Advanced Materials), and the like
(Vinylimidazolium trichloride/vinylpyrrolidone copolymer)
Polyquaternium-16: Luviquat FC370 (manufactured by BASF), and the like

The component (C) is preferably cationized hydroxyalkyl cellulose, cationized guar gum, a diallyldimethylammonium chloride/acrylamide copolymer, and an N,N-dimethylaminoethyl methacrylate diethyl sulfate/N,N-dimethylacrylamide/polyethylene glycol dimethacrylate copolymer, from a viewpoint of allowing PIC to adsorb onto the film of foams and forming dense foams capable of retaining carbon dioxide gas, thereby providing uniform foam texture, good foam retention, and excellent lastingness during massage.

One or more of the component (C) can be used, and the content in the stock solution is preferably 0.01 mass% or more, more preferably 0.02 mass% or more, and further more preferably 0.04 mass% or more, and preferably 3 mass% or less, more preferably 2 mass% or less, and further preferably 1 mass% or less, from a viewpoint of allowing PIC to adsorb onto the film of foams and forming dense foams capable of retaining carbon dioxide gas, thereby providing uniform foam texture, good foam retention, and excellent lastingness during massage. The content of the component (C) in the stock solution is preferably from 0.01 to 3 mass%, more preferably from 0.02 to 2 mass%, and further preferably from 0.04 to 1 mass%.

In the present invention, the mass ratio of the component (A) to a total of the components (B) and (C), (A)/((B)+(C)), is 6 or more, preferably 7 or more, and more preferably 8 or more, and preferably 250 or less, more preferably 220 or less, further more preferably 50 or less, and even more preferably 30 or less, from a viewpoint of uniform texture of the discharged foam, good foam retention, and excellent lastingness during massage, and further retaining a feel of moist skin after washing.

The mass ratio of the component (A) to a total of the components (B) and (C), (A)/((B)+(C)), is 6 or more, preferably from 6 to 250, more preferably from 7 to 220, further more preferably from 7 to 50, and even more preferably from 8 to 30.

In the present invention, the mass ratio of the component (B) to the component (C), (B)/(C), is preferably 0.2 or more, more preferably 0.4 or more, further more preferably 0.6 or more, and even more preferably 0.8 or more, and preferably 4 or less, more preferably 3 or less, further more preferably 2 or less, and even more preferably 1.5 or less, from a viewpoint of uniform texture of the discharged foam, good foam retention, excellent lastingness during massage, and further retaining a feel of moist skin after washing. The mass ratio of the component (B) to the component (C), (B)/(C), is preferably from 0.2 to 4, more preferably from 0.4 to 3, further more preferably from 0.6 to 2, and even more preferably from 0.8 to 1.5.

The aerosol cosmetic of the present invention can further contain, in the stock solution, polyhydric alcohol, and such a cosmetic has good foam retention and excellent use impression after application. The polyhydric alcohol is a compound having 2 or more hydroxyl groups in a molecule.

Examples of the dihydric alcohol include ethylene glycol, diethylene glycol, triethylene glycol, polyethylene glycol, propylene glycol, dipropylene glycol, tripropylene glycol, polypropylene glycol, 1,3-butylene glycol, and 1,3-propanediol. Examples of the trihydric alcohol include glycerin, and trimethylolpropane. Examples of the tetrahydric alcohol include diglycerin, and erythritol. Examples of the pentahydric or higher polyhydric alcohols include polyglycerin such as triglycerin; sugars and sugar alcohols such as glucose, maltose, maltitol, sucrose, xylitol, sorbitol, malbitol, polyoxyethylene methyl glucoside, polyoxyethylene ethyl glucoside, and polyoxyethylene propylene glucoside.

Of these, it is preferable to contain one or more selected from the group consisting of 1,3-butylene glycol and polyoxyethylene methyl glucoside, from a viewpoint of uniform texture of the discharged foam, good foam retention, and excellent lastingness during massage.

One or more kind of polyhydric alcohol can be used, and the content in the stock solution is preferably 0.5 mass% or more, more preferably 1 mass% or more, and further more preferably 3 mass% or more, and preferably 40 mass% or less, more preferably 30 mass% or less, and further preferably 20 mass% or less, from a viewpoint of good foam retention, and excellent use impression after application. The content of polyhydric alcohol in the stock solution is preferably from 0.5 to 40 mass%, more preferably from 1 to 30 mass%, and further preferably from 3 to 20 mass%.

The aerosol cosmetic of the present invention further contains, in the stock solution, water. Water works as the solvent of the stock solution and composes the balance of the other components. The content of water in the stock solution is preferably from 30 to 85 mass%, more preferably from 40 to 80 mass%, and further preferably from 50 to 75 mass%, from a viewpoint of enhancing the foam retention, and excellent use impression.

In the aerosol cosmetic of the present invention, the stock solution can further contain components used in typical cosmetics such as a surfactant other than the component (A), an oil component, ethanol, a preservative, an antioxidant, a pigment, a pH regulator, a perfume, an UV absorber, a moisturizer, a blood circulation promoter, a cooling agent, an antiperspirant, a disinfectant, a whitening agent, an anti-inflammatory agent, and a skin activator.

In the aerosol cosmetic of the present invention, the stock solution is prepared by mixing the components (A), (B) and (C), and other components. The stock solution has a viscosity at 25°C of preferably from 400 to 10 000 mPa·s, more preferably from 500 to 8 500 mPa·s, and further preferably from 500 to 7 000 mPa·s, from a viewpoint of being an appropriate viscosity for excellent foam massage properties.

The viscosity herein is a value measured using a BM viscometer (manufactured by Toki Sangyo Co., Ltd.), with No. 3 rotor at 12 rpm for 1 min, except that it is measured with No. 2 rotor at 12 rpm for 1 min, for a viscosity of more than 9 000 mPa·s, whereas it is measured with No. 4 rotor at 100 rpm for 1 min, for a viscosity of less than 100 mPa·s.

The aerosol cosmetic of the present invention can be produced by filling the stock solution described above, and (D) the carbon dioxide gas into a pressure resistant container. The mode of spraying is preferably a foam type by which the aerosol cosmetic is discharged in a foam form.

The mass ratio of the stock solution to the carbon dioxide gas is preferably from 94:6 to 99.5:0.5, more preferably from 95:5 to 99:1, and further more preferably from 96.5:3.5 to 98.5:1.5, from a viewpoint of excellent foam generation from carbon dioxide gas, and retaining a feel of moist skin after application.

The aerosol cosmetic of the present invention can contain a propellant used in typical aerosol cosmetics, other than the carbon dioxide gas of the component (D). Examples of the propellant include liquefied petroleum gases and compressed gases. When a propellant other than the carbon dioxide gas is contained, the content of the carbon dioxide gas in relative to a total amount of the carbon dioxide gas and the propellant is preferably 85 mass% or more, more preferably 90 mass% or more, further more preferably 95 mass% or more, and even more preferably 98 mass% or more.

In the present invention, the aerosol cosmetic preferably does not contain a propellant other than the carbon dioxide gas, or when the aerosol cosmetic contains a propellant other than the carbon dioxide gas, the content of the carbon dioxide gas relative to a total amount of the carbon dioxide gas and the propellant is preferably 85 mass% or more.

The aerosol cosmetic of the present invention can be applied as a cosmetic without particular limitation, and is suitable as a skin cosmetic, from a viewpoint of reducing the volatilization of the carbon dioxide gas in the cosmetic after it is sprayed, and a high effect of the carbon dioxide gas on the skin after application due to a high concentration of the carbon dioxide gas.

The aerosol cosmetic of the present invention can be used in accordance with use situation such as being applied to the skin, left for a certain period of time, allowed to settle on the skin, massaged, wiped or washed off.

The aerosol cosmetic of the present invention can be produced by a typical method. For example, the aerosol cosmetic can be produced by including the following steps 1 to 3.

Step 1: a step of dispersing components including the components (A) and (B) (stock solution components other than the component (C)) using a disperser, and then heating them to 50°C or more to mix the components.

Step 2: a subsequent step of adding the component (C) and mixing the resultant using a propeller to prepare a stock solution.

Step 3: a step of cooling the stock solution obtained in the step 2 to 15 to 30°C, then filling the stock solution into a pressure resistant container, hermetically sealing the container, and thereafter filling the component (D) under pressure.

Thus, when preparing the stock solution, it is considered that when the cationic polymer of the component (C) is added after the anionic polymer of the component (B) is neutralized, PIC to be formed is likely to be small. This forms uniform foam texture, thereby enhancing the foam retention performance.

Regarding the embodiments described above, the present invention further discloses the following compositions, and the like.

<1> An aerosol cosmetic comprising a stock solution comprising:
the following components (A), (B), and (C),

(A) an anionic surfactant;
(B) an anionic polymer; and
(C) a cationic polymer having a cationic charge density of 5.0 meq/g or less,
a mass ratio of the component (A) to a total of the components (B) and (C), (A)/((B)+(C)), is 6 or more, and
(D) carbon dioxide gas.

<2> The aerosol cosmetic according to <1>, wherein the component (A) is preferably one or more selected from the group consisting of fatty acids having 12 to 22 carbon atoms, or salts thereof, alkyl sulfate esters having 12 to 22 carbon atoms, or salts thereof, polyoxyethylene alkyl ether sulfates having 12 to 22 carbon atoms, or salts thereof, polyoxyethylene alkyl ether phosphates having 12 to 22 carbon atoms, or salts thereof, dialkyl sulfosuccinic acids having 12 to 24 carbon atoms, or salts thereof, N-alkyloyl methyl taurines having 12 to 22 carbon atoms, or salts thereof, and N-acyl glutamic acids having 12 to 22 carbon atoms, or salts thereof, more preferably polyoxyethylene alkyl ether sulfates, or salts thereof, and N-acyl glutamic acids having 12 to 22 carbon atoms, or salts thereof, and further preferably polyoxyethylene lauryl ether sulfate, or a salt thereof, and N-acyl glutamic acid of coconut oil fatty acid, or a salt thereof.

<3> The aerosol cosmetic according to <1> or <2>, wherein a content of the component (A) in the stock solution is preferably 1 mass% or more, more preferably 3 mass% or more, and further more preferably 5 mass% or more, and preferably 40 mass% or less, more preferably 25 mass% or less, and further preferably 15 mass% or less.

<4> The aerosol cosmetic according to any one of <1> to <3>, wherein the component (B) is preferably an acrylic acid polymer, more preferably an acrylic acid/(meth)acrylic acid alkyl copolymer, a carboxyvinyl polymer, and a polyoxyethylene alkyl ether-modified acrylic acid copolymer, and further preferably comprises at least one selected from the group consisting of (acrylates/($C_{10-30}$) alkyl (meth) acrylate) copolymers, carboxyvinyl polymers, and (acrylates/steareth-20 methacrylate) copolymers.

<5> The aerosol cosmetic according to any one of <1> to <4>, wherein a content of the component (B) in the stock solution is preferably 0.01 mass% or more, more preferably 0.02 mass% or more, and further more preferably 0.04 mass% or more, and preferably 2 mass% or less, more preferably 1.5 mass% or less, and further preferably 1 mass% or less.

<6> The aerosol cosmetic according to any one of <1> to <5>, wherein the component (C) preferably has a cationic charge density of from 0.8 to 4 meq/g, and more preferably from 1 to 3.3 meq/g.

<7> The aerosol cosmetic according to any one of <1> to <6>, wherein the component (C) is more preferably one or more preferably selected from the group consisting of cationized hydroxyalkyl celluloses, cationized guar gums, diallyldimethylammonium chloride/acrylamide copolymers, N,N-dimethylaminoethyl methacrylate diethyl sulfate/N,N-dimethylacrylamide/polyethylene glycol dimethacrylate copolymers.

<8> The aerosol cosmetic according to any one of <1> to <7>, wherein a content of the component (C) in the stock solution is preferably 0.01 mass% or more, more preferably 0.02 mass% or more, and further more preferably 0.04 mass% or more, and preferably 3 mass% or less, more preferably 2 mass% or less, and further preferably 1 mass% or less.

<9> The aerosol cosmetic according to any one of <1> to <8>, wherein a mass ratio of the component (A) to a total of the components (B) and (C), (A)/((B)+(C)), is preferably 7 or more, and more preferably 8 or more, and preferably 250 or less, more preferably 220 or less, further more preferably 50 or less, and even more preferably 30 or less.

<10> The aerosol cosmetic according to any one of <1> to <9>, wherein a mass ratio of the component (B) to the component (C), (B)/(C), is preferably 0.2 or more, more preferably 0.4 or more, further more preferably 0.6 or more, and even more preferably 0.8 or more, and preferably 4 or less, more preferably 3 or less, further more preferably 2 or less, and even more preferably 1.5 or less.

<11> The aerosol cosmetic according to any one of <1> to <10>, wherein the stock solution further preferably comprises polyhydric alcohol.

<12> The aerosol cosmetic according to <11>, wherein the polyhydric alcohol is preferably one or more selected from the group consisting of 1,3-butylene glycol, and polyoxyethylene methyl glucoside.

<13> The aerosol cosmetic according to <11> or <12>, wherein a content of the polyhydric alcohol in the stock solution is preferably 0.5 mass% or more, more preferably 1 mass% or more, and further more preferably 3 mass% or more, and preferably 40 mass% or less, more preferably 30 mass% or less, and further preferably 20 mass% or less.

<14> The aerosol cosmetic according to any one of <1> to <13>, wherein a content of water in the stock solution is preferably from 30 to 85 mass%, more preferably from 40 to 80 mass%, and further preferably from 50 to 75 mass%.

<15> The aerosol cosmetic according to any one of <1> to <14>, wherein the stock solution has a viscosity at 25°C of preferably from 400 to 10 000 mPa·s, more preferably from 500 to 8 500 mPa·s, and further preferably from 500 to 7 000 mPa·s.

<16> The aerosol cosmetic according to any one of <1> to <15>, produced by filling the stock solution and (D) the carbon dioxide gas into a pressure resistant container, wherein a mass ratio of the stock solution to the carbon dioxide gas is preferably from 94:6 to 99.5:0.5, more preferably from 95:5 to 99:1, and further preferably from 96.5:3.5 to 98.5:1.5.

<17> The aerosol cosmetic according to any one of <1> to <16>, wherein the aerosol cosmetic preferably does not contain a propellant other than the carbon dioxide gas, or when the aerosol cosmetic contains a propellant other than the carbon dioxide gas, the content of the carbon dioxide gas relative to a total amount of the carbon dioxide gas and the propellant is preferably 85 mass% or more, more preferably 90 mass% or more, further more preferably 95 mass% or more, and even more preferably 98 mass% or more.

<18> The aerosol cosmetic according to any one of <1> to <17>, wherein the form of spray is preferably a foam type by which the aerosol cosmetic is discharged in a foam form.

<19> The aerosol cosmetic according to any one of <1> to <18>, being a skin cosmetic.

<20> The aerosol cosmetic according to any one of <1> to <19>, which is used by being applied to the skin, left for a certain period of time, allowed to settle on the skin, massaged, wiped or washed off.

<21> A method for producing the aerosol cosmetic according to any one of <1> to <20>, comprising the following steps 1 to 3:

step 1: a step of stirring components including the components (A) and (B), and heating them to 50°C or more to mix the components,
step 2: a subsequent step of adding the component (C) and stirring and mixing the components to prepare a stock solution, and

step 3: a step of cooling the stock solution obtained in the step 2, then filling the stock solution into a pressure resistant container, hermetically sealing the container, and thereafter filling the component (D) under pressure.

Example

Examples 1 to 8 and Comparative Examples 1 to 3

The aerosol cosmetics having the compositions shown in Table 1 were produced, and the particle size of discharged foam was measured, and the uniformity in foam texture, the foam retention (lastingness during massage), and the retention of a moist feel after washing (Δ moisture content) were also evaluated. The results are collectively shown in Table 1.

(Production method)

Step 1: Components including the components (A) and (B) were dispersed using a disperser, and then heated to 60°C to 70°C to mix them.

Step 2: Subsequently, the component (C) was added and the resultant was mixed using a propeller to prepare a stock solution.

Step 3: The stock solution obtained in step 2 was cooled to 15 to 30°C, then filled into a pressure resistant container, which was hermetically sealed, and thereafter the component (D) was filled under pressure to obtain an aerosol cosmetic. 2.1 parts by mass of carbon dioxide was blended into 97.9 parts by mass of the stock solution (stock solution : carbon dioxide gas = 97.9 : 2.1)

(Evaluation methods)

(1) (Foam particle size)/(standard deviation) (μm):

The foam of each cosmetic was discharged onto a slide glass, a cover glass was gently placed thereon without pressing down, a photograph of the foam 10 seconds after discharged was taken using a digital microscope (KH-8700, manufactured by HYROX, 100x magnification). The diameters of all the foams present in an area of 500 μm ϕ near the center of the obtained image were measured. During the measurement, when the number of foams in this area was less than 50 foams, the measurement was carried out using the foams in the adjacent area so that the total number reached 50 foams.

The results are shown in the average particle size and the standard deviation.

(2) Uniformity in foam texture:

The foam of each cosmetic was discharged onto a slide glass, a cover glass was gently placed thereon without pressing down, a photograph of the foam 10 seconds after discharged was taken using a digital microscope (KH-8700, manufactured by HYROX, 100x magnification). The obtained images were evaluated visually on the following 5 scales by professional evaluators.

5: Foam sizes are uniform.
4: Foam sizes are slightly uniform.
3: Foam sizes are slightly ununiform.
2: Foam sizes are ununiform.
1: Foam sizes are considerably ununiform.

(3) Foam retention (Lastingness during massage):

Professional evaluators discharged the foam of each cosmetic on the back of the hand, applied a shear force for 5 seconds in such a way as to draw circles with finger tips, and then a feel of foam thickness was evaluated on the following 5 scales.

5: A feel of foam thickness is well massageable.

4: A feel of foam thickness is massageable.
3: A feel of foam thickness is slightly massageable.
2: A feel of foam thickness is not much massageable.
1: A feel of foam thickness is not massageable.

(4) Retention of a moist feel after washing ($\Delta$ moisture content):

The moisture content of the forearm was measured 5 times using Corneometer CM825 (manufactured by Courage+Khazaka electronic GmbH) to determine the average value. Onto the measurement site, the foam of each cosmetic was discharged to massage for 5 seconds, then rinsed off with tap water, and towel dried. Fifteen minutes after washing, the moisture content of the forearm was measured again 5 times using Corneometer to determine the average value. The results show the moisture contents before and after washing, and the difference therebetween ($\Delta$ moisture content; [(moisture content 15 minutes after washing) - (moisture content before washing)]).

[Table 1]

| | | | Example 1 | Example 2 | Example 3 | Example 4 | Example 5 | Example 6 | Example 7 | Example 8 | Comparative Example 1 | Comparative Example 2 | Comparative Example 3 |
|---|---|---|---|---|---|---|---|---|---|---|---|---|---|
| | | <Stock solution> | 97.900 | 97.900 | 97.900 | 97.900 | 97.900 | 97.900 | 97.900 | 97.900 | 97.900 | 97.900 | 97.900 |
| Step 1 | (A) | Sodium N-coconut oil fatty acid acyl-L-glutamate | 3.500 | 3.500 | 3.500 | 3.500 | 3.500 | 3.500 | 3.500 | 3.500 | 3.500 | 3.500 | 2.500 |
| | | Sodium polyoxyethylene(2) lauryl ether sulfate (∗ active 27%) | 16.670 | 16.670 | 16.670 | 16.670 | 16.670 | 16.670 | 16.670 | 16.670 | 16.670 | 16.670 | 1.800 |
| | (B) | Acrylic acid·methacrylic acid alkyl copolymer (manufactured by Lubrizol Advanced Materials, Pemulen TR-1) | 0.400 | 0.250 | 0.600 | | 0.400 | 0.400 | 0.400 | 0.400 | 0.400 | 0.400 | 0.300 |
| | | Carboxyvinyl polymer (manufactured by Lubrizol Advanced Materials, Carbopol 981) | | | | 0.400 | | | | | | | |
| Step 2 | (C) | Cationized hydroxyethyl cellulose (cationic charge density 1.1 meq/g) (manufactured by Kao Corporation, Caticello M-80) | 0.300 | 0.300 | 0.300 | 0.300 | 0.200 | 0.500 | | | | | 0.300 |
| | | Polyquaternium-7 (cationic charge density 3.1 meq/g) (manufactured by Lubrizol Advanced Materials, MERQUAT 550 (∗ active 9%)), | | | | | | | 3.333 | | | | |
| | | Cationized guar gum (cationic charge density 1.8 meq/g) (manufactured by Solvay Rhodia, Jaguar C-17) | | | | | | | | 0.300 | | | |
| | Other components | Polyquaternium-22 (cationic charge density 6.0 meq/g) (manufactured by Lubrizol Advanced Materials, MERQUAT 295 (∗ active 37%)) | | | | | | | | | 0.811 | | |
| Step 1 | | Hydroxyethyl cellulose (manufactured by Daicel Corporation, HEC Daicel SE850) | | | | | | | | | | 0.300 | |
| | | 1,3-Butylene glycol | 5.000 | 5.000 | 5.000 | 5.000 | 5.000 | 5.000 | 5.000 | 5.000 | 5.000 | 5.000 | 5.000 |
| | | Polyoxyethylene methyl glucoside | 10.000 | 10.000 | 10.000 | 10.000 | 10.000 | 10.000 | 10.000 | 10.000 | 10.000 | 10.000 | 10.000 |
| | | Myristyl alcohol | 0.300 | 0.300 | 0.300 | 0.300 | 0.300 | 0.300 | 0.300 | 0.300 | 0.300 | 0.300 | 0.300 |
| | | 48% Sodium hydroxide | 0.300 | 0.200 | 0.450 | 0.300 | 0.300 | 0.300 | 0.300 | 0.300 | 0.300 | 0.300 | 0.200 |
| | | Phenoxyethanol | 0.450 | 0.450 | 0.450 | 0.450 | 0.450 | 0.450 | 0.450 | 0.450 | 0.450 | 0.450 | 0.450 |
| | | Disodium edetate | 0.200 | 0.200 | 0.200 | 0.200 | 0.200 | 0.200 | 0.200 | 0.200 | 0.200 | 0.200 | 0.200 |

| | | | | | | | | | | | | | |
|---|---|---|---|---|---|---|---|---|---|---|---|---|---|
| | | Purified water | 62.880 | 63.130 | 62.530 | 62.880 | 62.980 | 62.680 | 59.847 | 62.880 | 62.369 | 62.880 | 78.950 |
| | | | 100.000 | 100.000 | 100.000 | 100.000 | 100.000 | 100.000 | 100.000 | 100.000 | 100.000 | 100.000 | 100.000 |
| | | \<Propellant\> | 2.100 | 2.100 | 2.100 | 2.100 | 2.100 | 2.100 | 2.100 | 2.100 | 2.100 | 2.100 | 2.100 |
| | (D) | Carbon dioxide | 100.000 | 100.000 | 100.000 | 100.000 | 100.000 | 100.000 | 100.000 | 100.000 | 100.000 | 100.000 | 100.000 |
| | | (A) Total amount | 8.00 | 8.00 | 8.00 | 8.00 | 8.00 | 8.00 | 8.00 | 8.00 | 8.00 | 8.00 | 2.99 |
| | | (B) Total amount | 0.40 | 0.25 | 0.60 | 0.40 | 0.40 | 0.40 | 0.40 | 0.40 | 0.40 | 0.40 | 0.30 |
| | | (C) Total amount | 0.30 | 0.30 | 0.30 | 0.30 | 0.20 | 0.50 | 0.30 | 0.30 | - | - | 0.30 |
| | | (A)/((B)+(C)) | 11.43 | 14.55 | 8.89 | 11.43 | 13.33 | 8.89 | 11.43 | 11.43 | - | - | 4.98 |
| | | (B)/(C) | 1.33 | 0.83 | 2.00 | 1.33 | 2.00 | 0.80 | 1.33 | 1.33 | - | - | 1.00 |
| Evaluations | | Uniformity in foam texture | 5 | 4 | 5 | 4 | 5 | 5 | 5 | 4 | 2 | 2 | 3 |
| | | Particle size of foam: average particle size ($\mu$m) | 61 | 96 | 68 | 76 | 71 | 56 | 56 | 70 | 80 | 81 | 69 |
| | | Standard deviation ($\mu$m) | 30 | 30 | 27 | 27 | 26 | 24 | 23 | 30 | 53 | 54 | 47 |
| | | Foam retention (lastingness during massage) | 5 | 4 | 5 | 5 | 4 | 5 | 5 | 4 | 1 | 2 | 1 |
| | | Retention of a moist feel after washing ($\Delta$ moisture content; 15 minutes after washing - before washing) | 2.7 | 1.5 | 2.7 | 3.0 | 3.8 | 3.8 | 3.1 | 0.8 | -3.5 | -0.3 | -2.8 |
| | | moisture content (before washing) | 25.7 | 30.7 | 31.0 | 30.3 | 28.4 | 29.9 | 28.4 | 29.3 | 33.7 | 32.5 | 35.2 |
| | | moisture content (15 minutes after washing) | 28.4 | 32.2 | 33.7 | 33.3 | 32.2 | 33.7 | 31.5 | 30.2 | 30.2 | 32.2 | 32.4 |

Example 9

As with Examples 1 to 8, an aerosol cosmetic having the composition shown in Table 2 was produced.
The obtained aerosol cosmetic had uniform texture of the discharged foam, good foam retention, and excellent lastingness during massage. Additionally, a feel of moist skin after washing retained.

[Table 2]

| | | | | Example |
|---|---|---|---|---|
| | | | | 9 |
| <Stock solution> | | | | 97.90 |
| Step 1 | (A) | | Sodium N-coconut oil fatty acid acyl-L-glutamate | 3.00 |
| | | | Sodium polyoxyethylene(2) lauryl ether sulfate (* active 27%) | 15.00 |
| | (B) | | Acrylic acid-methacrylic acid alkyl copolymer (manufactured bv Lubrizol Advanced Materials, Pemulen TR-1) | 0.35 |
| Step 2 | (C) | | Cationized hydroxyethyl cellulose (cationic charge density 1.1 meq/g) (manufactured bv Kao Corporation, Caticello M-80) | 0.25 |
| Step 1 | Other components | | Hydroxyethyl cellulose | 0.15 |
| | | | 1,3-Butylene glycol | 5.00 |
| | | | Polyoxyethylene methyl glucoside | 10.00 |
| | | | Myristyl alcohol | 0.80 |
| | | | Sodium hydroxide | 0.15 |
| | | | Phenoxyethanol | 0.50 |
| | | | Disodium edetate | 0.20 |
| | | | Perfume | 0.20 |
| | | | Purified water | 64.40 |
| | | | | 100.00 |
| <Propellant> | | | | 2.10 |
| (D) | | Carbon dioxide | | 100.00 |
| (A) Total amount | | | | 7.05 |
| (B) Total amount | | | | 0.35 |
| (C) Total amount | | | | 0.25 |
| (A)/((B)+(C)) | | | | 11.75 |
| (B)/(C) | | | | 1.40 |

Examples 10 to 17 and Comparative Example 4

As with Examples 1 to 9, the aerosol cosmetics having the compositions shown in Table 3 were produced. The particle size of discharged foam was measured, and the uniformity in foam texture, the foam retention (lastingness during massage), and the retention of a moist feel after washing ($\Delta$ moisture content) were also evaluated. The results are collectively shown in Table 3.

12

[Table 3]

|  |  |  | Example 10 | Example 11 | Example 12 | Example 13 | Example 14 | Example 15 | Example 16 | Example 17 | Comparative Example 4 |
|---|---|---|---|---|---|---|---|---|---|---|---|
| | | <Stock solution> | 97.900 | 97.900 | 97.900 | 97.900 | 97.900 | 97.900 | 97.900 | 97.900 | 97.900 |
| Step 1 | (A) | Sodium N-coconut oil fatty acid acyl-L-glutamate | 3.500 | 3.500 | 3.500 | 3.500 | | 22.000 | 3.500 | 12.000 | 3.500 |
| | | Sodium polyoxyethylene (2) lauryl ether sulfate (* active 27%) | 16.670 | 16.670 | 16.670 | 16.670 | 5.556 | 37.037 | 16.670 | 16.670 | 16.670 |
| | (B) | Acrylic acid·methacrylic acid alkyl copolymer (manufactured by Lubrizol Advanced Materials, Pemulen TR-1) | 0.400 | 0.060 | | 0.400 | 0.020 | 0.400 | 0.020 | 0.800 | |
| | | (Acrylates/steareth-20 methacrylate) copolymer (manufactured by The Dow Chemical Company, Aculyn 22 (* active 30%)) | | | 1.333 | | | | | | |

EP 4 342 443 A1

13

(continued)

| | | | Example 10 | Example 11 | Example 12 | Example 13 | Example 14 | Example 15 | Example 16 | Example 17 | Comparative Example 4 |
|---|---|---|---|---|---|---|---|---|---|---|---|
| Step 2 | (C) | Cationized hydroxyethyl cellulose (cationic charge density 1.1 meq/g) (manufactured by Kao Corporation, Caticello M-80) | 0.300 | 0.300 | 0.300 | | 0.020 | 0.300 | 0.020 | 1.500 | 0.300 |
| | | Polyquaternium-52: (cationic charge density 0.83 meq/g) (manufactured by Kao Corporation, Sofcare KG-101W-E (* active 2.4%)) | | | | 12.500 | | | | | |

| | | | Example 10 | Example 11 | Example 12 | Example 13 | Example 14 | Example 15 | Example 16 | Example 17 | Comparative Example 4 | |
|---|---|---|---|---|---|---|---|---|---|---|---|---|
| Step 1 | Other components | Hydroxyethyl cellulose (manufactured by Daicel Corporation, HEC Daicel SE850) | | 0.200 | 0.200 | 0.200 | 1.000 | 0.100 | 1.000 | | | |
| | | 1,3-Butylene glycol | 5.000 | 5.000 | 5.000 | 5.000 | 5.000 | 1.000 | 5.000 | 5.000 | 5.000 | |
| | | Polyoxyethylene methyl glucoside | 10.000 | 10.000 | 10.000 | 10.000 | 10.000 | | 10.000 | 10.000 | 10.000 | |
| | | Myristyl alcohol | 0.300 | 0.300 | 0.300 | 0.300 | 0.300 | 0.300 | 0.300 | 0.300 | 0.300 | |
| | | 48% Sodium hydroxide | 0.300 | 0.500 | 0.300 | 0.300 | 0.100 | 0.300 | 0.200 | 0.700 | 0.100 | |
| | | Phenoxyethanol | 0.450 | 0.450 | 0.450 | 0.450 | 0.450 | 0.450 | 0.450 | 0.450 | 0.450 | |
| | | Disodium edetate | 0.200 | 0.200 | 0.200 | 0.200 | 0.200 | 0.200 | 0.200 | 0.200 | 0.200 | |
| | | Purified water | 62.880 | 62.820 | 61.747 | 50.480 | 77.354 | 37.913 | 62.640 | 52.380 | 63.480 | |
| | | | 100.000 | 100.000 | 100.000 | 100.000 | 100.000 | 100.000 | 100.000 | 100.000 | 100.000 | |
| Viscosity (mPa·s) | | | 420 | 780 | 790 | 880 | 4820 | 410 | 7120 | 9100 | 48 | |
| <Propellant> | | | 2.100 | 2.100 | 2.100 | 2.100 | 2.100 | 2.100 | 2.100 | 2.100 | 2.100 | |
| (D) | | Carbon dioxide | 90.000 | 100.000 | 100.000 | 100.000 | 100.000 | 100.000 | 100.000 | 100.000 | 100.000 | |
| | | LPG | 10.000 | | | | | | | | | |
| (A) Total amount | | | 8.00 | 8.00 | 8.00 | 8.00 | 1.50 | 32.00 | 8.00 | 16.50 | 8.00 | |
| (B) Total amount | | | 0.40 | 0.06 | 0.40 | 0.40 | 0.02 | 0.40 | 0.02 | 0.80 | 0.00 | |
| (C) Total amount | | | | 0.30 | 0.30 | 0.30 | 0.30 | 0.02 | 0.30 | 0.02 | 1.50 | 0.30 |
| (A)/((B)+(C)) | | | | 11.43 | 22.22 | 11.43 | 11.43 | 37.50 | 45.71 | 200.02 | 7.17 | 26.67 |
| (B)/(C) | | | | 1.33 | 0.20 | 1.33 | 1.33 | 1.00 | 1.33 | 1.00 | 0.53 | 0.00 |

(continued)

| Evaluations | | Example 10 | Example 11 | Example 12 | Example 13 | Example 14 | Example 15 | Example 16 | Example 17 | Comparative Example 4 | |
|---|---|---|---|---|---|---|---|---|---|---|---|
| | Uniformity in foam texture | 4 | 4 | 4 | 5 | 5 | 4 | 5 | 5 | 1 | |
| | Particle size of foam: average particle size ($\mu$m) | 67 | 92 | 75 | 77 | 57 | 65 | 62 | 62 | 95 | |
| | Standard deviation ($\mu$m) | 32 | 36 | 39 | 29 | 26 | 33 | 28 | 26 | 74 | |
| | Foam retention (lastingness during massage) | 4 | 4 | 5 | 4 | 5 | 5 | 5 | 5 | 1 | |
| | Retention of a moist feel after washing ($\Delta$ moisture content; 15 minutes after washing - before washing) | 2.2 | 0.8 | 2.2 | 1.6 | 5.3 | 5.0 | 4.5 | 5.1 | -1.9 | |
| | moisture content (before washing) | 29.8 | 35.1 | 34.0 | 30.1 | 31.5 | 31.1 | 29.4 | 31.9 | 32.3 | |
| | moisture content 15 minutes after washing) | 32.0 | 36.0 | 36.2 | 31.7 | 36.8 | 36.1 | 33.9 | 37.0 | 30.4 | |

**Claims**

1.  An aerosol cosmetic comprising a stock solution comprising:
    the following components (A), (B), and (C),

    (A) an anionic surfactant;
    (B) an anionic polymer; and
    (C) a cationic polymer having a cationic charge density of 5.0 meq/g or less,
    a mass ratio of the component (A) to a total of the components (B) and (C), (A)/((B)+(C)), being 6 or more, and
    (D) carbon dioxide gas.

2.  The aerosol cosmetic according to claim 1, wherein the component (A) is one or more selected from the group consisting of fatty acids having 12 to 22 carbon atoms, or salts thereof, alkyl sulfate esters having 12 to 22 carbon atoms, or salts thereof, polyoxyethylene alkyl ether sulfates having 12 to 22 carbon atoms, or salts thereof, polyoxyethylene alkyl ether phosphates having 12 to 22 carbon atoms, or salts thereof, dialkyl sulfosuccinic acids having 12 to 24 carbon atoms, or salts thereof, N-alkyloyl methyl taurines having 12 to 22 carbon atoms, or salts thereof, and N-acyl glutamic acids having 12 to 22 carbon atoms, or salts thereof.

3.  The aerosol cosmetic according to claim 1 or 2, wherein the component (B) is an acrylic acid polymer.

4.  The aerosol cosmetic according to any one of claims 1 to 3, wherein the component (C) is one or more selected from the group consisting of cationized hydroxyalkyl celluloses, diallyldimethylammonium chloride/acrylamide copolymers, N,N-dimethylaminoethyl methacrylate diethyl sulfate/N,N-dimethyl acrylamide/polyethylene glycol dimethacrylate copolymers, and cationized guar gums.

5.  The aerosol cosmetic according to any one of claims 1 to 4, wherein a content of the component (A) in the stock solution is from 1 to 40 mass%.

6.  The aerosol cosmetic according to any one of claims 1 to 5, wherein a content of the component (B) in the stock solution is from 0.01 to 2 mass%.

7.  The aerosol cosmetic according to any one of claims 1 to 6, wherein a content of the component (C) in the stock solution is from 0.01 to 3 mass%.

8.  The aerosol cosmetic according to any one of claims 1 to 7, wherein a mass ratio of the component (B) to the component (C), (B)/(C), is from 0.2 to 4.

9.  The aerosol cosmetic according to any one of claims 1 to 8, wherein a mass ratio of the stock solution/carbon dioxide gas is from 94:6 to 99.5:0.5.

10. The aerosol cosmetic according to any one of claims 1 to 9, wherein the aerosol cosmetic does not contain a propellant other than the carbon dioxide gas, or when the aerosol cosmetic contains a propellant other than the carbon dioxide gas, the content of the carbon dioxide gas relative to a total amount of the carbon dioxide gas and the propellant is 85 mass% or more.

11. The aerosol cosmetic according to any one of claims 1 to 10, wherein the stock solution further comprises a polyhydric alcohol.

12. The aerosol cosmetic according to claim 11, wherein a content of the polyhydric alcohol in the stock solution is 0.5 mass% or more.

13. The aerosol cosmetic according to any one of claims 1 to 12, wherein a content of water in the stock solution is from 30 to 85 mass%.

14. The aerosol cosmetic according to any one of claims 1 to 13, wherein a viscosity of the stock solution at 25°C is from 400 to 10 000 mPa·s.

15. A method for producing the aerosol cosmetic according to any one of claims 1 to 14, comprising the following steps 1 to 3:

step 1: a step of stirring components including the components (A) and (B), and heating them to 50°C or more to mix the components;

step 2: a subsequent step of adding the component (C), and stirring and mixing the components to prepare a stock solution; and

step 3: a step of cooling the stock solution obtained in the step 2, then filling the stock solution into a pressure resistant container, hermetically sealing the container, and thereafter filling the component (D) under pressure.

**EP 4 342 443 A1**

## INTERNATIONAL SEARCH REPORT

| International application No. |
|---|
| **PCT/JP2022/020410** |

**A. CLASSIFICATION OF SUBJECT MATTER**

*A61K 8/02*(2006.01)i; *A61K 8/19*(2006.01)i; *A61K 8/34*(2006.01)i; *A61K 8/44*(2006.01)i; *A61K 8/46*(2006.01)i; *A61K 8/73*(2006.01)i; *A61K 8/81*(2006.01)i; *A61Q 19/00*(2006.01)i

FI:  A61K8/02; A61K8/19; A61K8/34; A61K8/44; A61K8/46; A61K8/73; A61K8/81; A61Q19/00

According to International Patent Classification (IPC) or to both national classification and IPC

**B. FIELDS SEARCHED**

Minimum documentation searched (classification system followed by classification symbols)

A61K8/02; A61K8/19; A61K8/34; A61K8/44; A61K8/46; A61K8/73; A61K8/81; A61Q19/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

Mintel GNPD

**C. DOCUMENTS CONSIDERED TO BE RELEVANT**

| Category* | Citation of document, with indication, where appropriate, of the relevant passages | Relevant to claim No. |
|---|---|---|
| X | X Boost Mousse Wash, Rohto Pharmaceutical, November 2019, Mintel GNPD [online], [retrieved on 11 July 2022], Internet <URL: https://www.gnpd.com>, Accession No.7027923 in particular, product description, content of the appeal, component, product photos | 1-15 |
| X | Obagi（オバジ）オバジXブーストムースウォッシュ. ロート製薬. 31 August 2019 (date first available on Amazon.co.jp), Amazon.co.jp [online], [retrieved on 11 July 2022], Internet: <URL: https://www.amazon.co.jp/Obagi-オバジ-オバジX-ブーストムースウォッシュ-150g/dp/B07W5ZFVZP/ref=cm_cr_arp_d_product_top?ie=UTF8>, (Obagi Obagi X Boost Mousse Wash. Rohto Pharmaceutical.) in particular, description, from the manufacturer, customer reviews | 1-15 |
| A | WO 2019/098365 A1 (KAO CORP.) 23 May 2019 (2019-05-23) paragraph [0030] | 1-15 |
| A | JP 2013-241359 A (DAIZO CORP.) 05 December 2013 (2013-12-05) entire text | 1-15 |
| A | WO 2014/073245 A1 (MILBON CO., LTD.) 15 May 2014 (2014-05-15) entire text | 1-15 |

☐ Further documents are listed in the continuation of Box C.    ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 July 2022** | **26 July 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

19

INTERNATIONAL SEARCH REPORT
Information on patent family members

International application No.

**PCT/JP2022/020410**

| Patent document cited in search report | | | Publication date (day/month/year) | Patent family member(s) | | | Publication date (day/month/year) |
|---|---|---|---|---|---|---|---|
| WO | 2019/098365 | A1 | 23 May 2019 | US | 2020/0397680 | A1 | |
| | | | | paragraphs [0071]-[0083] | | | |
| | | | | EP | 3714870 | A1 | |
| | | | | CN | 111356437 | A | |
| | | | | TW | 201922215 | A | |
| | | | | JP | 2019-94326 | A | |
| JP | 2013-241359 | A | 05 December 2013 | (Family: none) | | | |
| WO | 2014/073245 | A1 | 15 May 2014 | JP | 2013-91642 | A | |
| | | | | KR | 10-2014-0059136 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010248098 A
- JP 2011084490 A
- JP S53139734 A
- JP S6036407 A